# EUROPEAN PATENT APPLICATION

(11) **EP 3 437 596 A1**
(43) Date of publication of application: **06.02.2019**
(21) Application number: 17775490.0
(22) Date of filing: 30.03.2017
(51) Int. Cl.: A61F 13/02

(54) **PATCH MATERIAL**

(30) Priority: 31.03.2016 US 201662315945 P
(71) Applicant: SENJU USA, INC., Woodland Hills, CA 91367 (US); Nichiban Co., Ltd., Bunkyo-ku Tokyo 112-8663 (JP)
(72) Inventor: OGAWA, Takahiro, Woodland Hills, California 91367 (US); ISOWAKI, Akiharu, Woodland Hills, California 91367 (US); OHTSU, Mariko, Tokyo 112-8663 (JP); HIRAOKA, Takao, Tokyo 112-8663 (JP); KENMOCHI, Chihiro, Tokyo 112-8663 (JP); MURATA, Atsushi, Tokyo 112-8663 (JP); KAWAHARA, Koji, Tokyo 112-8663 (JP)
(74) Representative: dompatent von Kreisler Selting Werner - Partnerschaft von Patent- und Rechtsanwälten mbB
(86) International application number: PCT/JP2017/013450
(87) International publication number: WO 2017/170956

(57) **Abstract**

The present invention addresses the problem of providing a patch material the ease of handling of which has been improved and with which it is easy to peel off a carrier layer that has been bonded to a support body layer after patching onto a site of application. The present invention is a patch product (1) composed of a patch material (2) and a take-up tape (7) covering an upper side thereof, wherein: the patch material (2) is constituted by being provided with an adhesive tape (10) substantially in the form of strip provided with a support body (4) and a pressure-sensitive adhesive layer (3) provided to the surface of one side of the support body (4), a release liner (5) that is affixed to the pressure-sensitive adhesive layer (3) and is more elongated than the support body (4), and a carrier film (6) that is bonded to the surface of the support body (4) on the side opposite the pressure-sensitive adhesive layer (3) side; the take-up tape (7) is provided with a tape support body (9) and a pressure-sensitive adhesive layer (8) provided on a surface thereof; the tape (7) is affixed to the carrier film (6), and preferably affixed to both the carrier film (6) and the release liner (5); and an end of one side of the carrier film (6) is not bonded to the surface of the support body (4) of the adhesive tape (10), thereby forming an unbonded end (6a) that is released from the support body (4).

## Description

### [TECHNICAL FIELD]

The present invention relates to an adhesive skin patch used in a medical and health field.

### [BACKGROUND ART]

Conventionally, there have been proposed various adhesive skin patches for human in a medical and health field. These adhesive skin patches basically comprise a laminated body of a plurality of layers, such as an adhesive layer, a backing film, and a release layer. The adhesive layer is applied to the skin. The backing film supports the adhesive layer. The release layer protects the adhesive layer until the adhesive layer is applied to the skin. Structures of respective layers are examined and selected by variously considering a purpose of use, an application site, a presence/absence of skin irritancy and uncomfortable feeling in applying to an applied surface (skin), an adherability to the skin, a followability when the skin expands and contracts and to a bending surface, and similar factors.

For example, there have been proposed an adhesive film and sheet (Patent Literature 1) in which a releasable lining layer constituted of a film material (a carrier layer), an adhesive layer and a base material is laminated on a surface on an opposite side of a side of an adhesive layer of a film material (a backing film) capable of being adapted to the skin. Furthermore, there have been proposed the adhesive film and sheet in which easy releasing of the film material (the carrier layer) from the film material (the backing film) capable of being adapted to the skin is available by releasing the releasable lining layer at the point of use.

Additionally, for example, there has been proposed an adhesive skin patch (Reference 2) in which a carrier sheet is brought into close contact with a surface of a flexible base material that includes an adhesive layer; and a bandage material (Reference 3) in which a backing film is disposed on a surface of a film (base material) that includes an adhesive layer and a grip strip is attached on the backing film.

### [PRIOR ART DOCUMENT]

### Patent Literature

Patent Literature 1: Japanese Patent Application Laid-Open No. 2007-020665
Patent Literature 2: Japanese Registered Utility Model No. 3187266
Patent Literature 3: U. S. Patent No. 6, 495, 230

### [SUMMARY OF INVENTION]

### Problems to be solved by Invention

In the conventionally proposed adhesive skin patches, for example, in order to reduce skin irritancy and uncomfortable feeling in applying to the skin, structuring each layer of an adhesive skin patch with thinner thickness and structuring the adhesive skin patch with a highly stretchable material have been employed. However, there have been issues that it is difficult to apply the above-structured adhesive skin patch to a desired place due to an easiness of bending when applied to the skin, and to apply the above-structured adhesive skin patch to the skin firmly due to such as having wrinkles in the adhesive skin patch when applied to the skin. Thus, when a handleability of the adhesive skin patch, especially the handleability of a backing film degrades, by disposing such as the above-described carrier layer in Patent Literature 1, the handleability of the backing film has been improved. The carrier layer is disposed on a surface on an opposite side of a side where an adhesive layer is disposed on the backing film, and attaching the backing film temporarily to the carrier layer can improve the handleability of the adhesive skin patch and an adherability to an applied surface.

However, the carrier layer itself needs to be thin to some extent in order to apply the adhesive skin patch according to a shape of an application site under the state that the carrier layer is temporarily attached to the backing film. In that case, it becomes a problem that a difficulty in releasing of a release layer when the adhesive skin patch is used due to a total thickness of the adhesive skin patch yet being thin, especially the difficulty in releasing of the carrier layer from the backing film after applying to the application site.

An object of the present invention is to provide an adhesive skin patch being allowed that a carrier layer being in close contact with a backing film layer is easily released after applying to an application site, so as to improve the handleability of the adhesive skin patch that comprises thin layers and to improve the handleability of the backing film layer.

### [MEANS TO SOLVE THE ABOVE PROBLEMS]

The inventors seriously studied in order to solve the above-described problems, and examined structures of an adhesive skin patch that achieve easy releasing of a carrier film (carrier layer) from a surface of a backing film, in addition to particularly achieving an improved handleability of the adhesive skin patch (easy releasing of a release liner and easy application of the adhesive skin patch).

As a result, in addition to that attaching a support liner on the carrier film ensures improving the handleability of the adhesive skin patch, such as easy releasing from the release liner and easy application to an application site when the adhesive skin patch is used, it was found that leaving a part of the carrier film in a state of being released from the surface of the backing film improves a difficulty in releasing the carrier film from the backing film. Thus, the present invention was completed.

The present invention relates to the following.

An adhesive skin patch product comprises an adhesive skin patch and a support liner that covers an upper side of the adhesive skin patch. The adhesive skin patch comprises an adhesive tape in a form of an approximately strip that includes a backing film and an adhesive layer disposed on a surface of one side of the backing film, a release liner that is attached to the adhesive layer and is longer than the backing film and a carrier film that is brought into close contact with a surface of the backing film that is opposite to a side of the adhesive layer.

Meanwhile, the support liner comprises a tape backing layer and a support adhesive layer disposed on a surface of the tape backing layer. The support liner is at least attached to the carrier film.

Further, the carrier film includes one end portion preventing close contact with the surface of the backing film of the adhesive tape, the one end portion being released from the backing film to form a non-attached end portion.

The present invention further provides the following embodiments.
[1] The adhesive skin patch product in which the support liner is attached to the carrier film and the release liner.
[2] The adhesive skin patch product in which the support liner is attached to the carrier film in a center of the support liner, and attached to the release liner in both ends of the support liner.
[3] The adhesive skin patch product in which the backing film of the adhesive tape is made of a polyolefin film, and the carrier film is made of a polyester film.
[4] The adhesive skin patch product in which the adhesive layer of the adhesive tape and the support adhesive layer of the support liner include a rubber-based adhesive.
[5] The adhesive skin patch product in which a proportion of an area of the non-attached end portion to a whole area of the carrier film is defined between 1% and 50%.

### [EFFECT OF INVENTION]

An adhesive skin patch product in the present invention employs a long release liner and a support liner and makes up a structure in which the support liner is attached on a carrier film. Accordingly, an adhesive tape becomes a pseudo-thickened tape and becomes easy to grasp, thereby enabling easy releasing of the adhesive tape from the release liner when an adhesive skin patch is used. Further, as described above, the adhesive tape that released the release liner becomes a thickened tape enabling easy grasping by attaching to the support liner through the carrier film. Therefore, easy application to an application site, especially easy application to the parts of the body that hardly come into sight and does not allow easy application, such as the face and the back of the body, can be improved.

Especially, in the adhesive skin patch product in the present invention, a structure in which the support liner is attached both to the carrier film and to the release liner (for example, when a longer tape than the carrier film is employed as a support liner), as well as grasping the support liner and releasing it from the release liner, and further grasping and pulling upward the support liner when the adhesive skin patch is used, ensure easier releasing of the adhesive tape from the release liner. In the present structure formed by attaching the long support liner through the carrier film, the adhesive tape that released the release liner becomes a pseudo-large-sized tape, and grasping the support liner as a handle enables a further improvement on easy application to the application site, especially easy application to the parts of the body that does not allow easy application, as described above.

Additionally, in the adhesive skin patch product in the present invention, forming a non-attached end portion that is out of close contact with a surface of a backing film in an end portion of the carrier film, as well as grasping and pulling upward the support liner from a side where the non-attached end portion is formed, ensure easier releasing of the carrier film from the backing film of the adhesive tape.

Furthermore, the adhesive skin patch product in the present invention can be made excellent in the hygiene aspect. In a particularly preferred aspect, the structure in which the support liner is attached both to the carrier film and to the release liner ensures an application to the application site without touching the backing film and an adhesive surface of the adhesive tape directly, thereby making the adhesive skin patch product even more hygienic.

### [BRIEF EXPLANATION OF DRAWING]

Fig. 1 is a sectional drawing illustrating one embodiment of the present invention and indicates a longitudinal direction of an adhesive skin patch product.
Fig. 2 is a sectional drawing illustrating one embodiment of the present invention and indicates a longitudinal direction of the adhesive skin patch product.

### [EMBODIMENTS CARRYING OUT THE PRESENT INVENTION]

The following further explains the present invention with the drawings illustrating preferable aspects of the present invention.

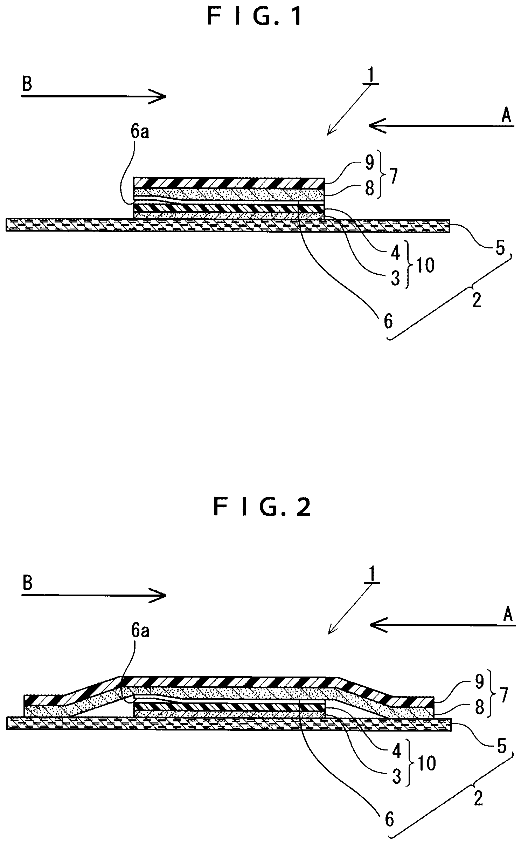

Fig. 1 is a drawing (a sectional drawing) illustrating one embodiment of an adhesive skin patch product in the present invention.

As illustrated in Fig. 1, an adhesive skin patch product 1 comprises an adhesive skin patch 2 and a support liner 7 that covers an upper side of the adhesive skin patch.

The adhesive skin patch 2 comprises an adhesive tape 10 provided with a backing film 4 and an adhesive layer 3 disposed on a surface of one side (lower side in Fig. 1) of the backing film 4, a release liner 5 that is attached to the adhesive layer 3 and is longer than the backing film 4 and a carrier film 6 that is brought into close contact with a surface of the backing film 4 that is opposite to a side of the adhesive layer 3.

Meanwhile, the support liner 7 comprises a tape backing layer 9 and a support adhesive layer 8 disposed on a surface of the tape backing layer 9. The support liner 7 is at least attached to the carrier film 6.

As illustrated in Fig. 1, the adhesive skin patch product 1 in the present invention has a feature in which the release liner 5 is longer than the adhesive tape 10 and the carrier film 6 that is brought into close contact with the surface of the backing film 4 of the adhesive tape 10, and the adhesive tape 10 and the carrier film 6 are disposed between the release liner 5 and the support liner 7, thereby having an enclosed structure. Thus, employing the release liner 5 that is longer than the adhesive tape 10 and attaching the support liner 7 to the adhesive tape 10 through the carrier film 6 make the adhesive tape 10 become a pseudo-thickened tape. Accordingly, even though a size of the adhesive tape 10 is small, easy grasping of the adhesive 10 and easy releasing of the adhesive 10 from the release liner 5 become available. Further, upon releasing from the release liner 5, easy application of the adhesive tape 10 to an application site improves, thereby ensuring an improved handleability of the adhesive skin patch.

Especially, the adhesive skin patch product 1 in the present invention has a feature in which, in an end portion on one side of the carrier film 6, a non-attached end portion 6a that is not in close contact with the surface of the backing film 4 and separated from the backing film 4 is formed. Thus, by arranging the non-attached end portion 6a, it triggers an improvement of easy releasing of the carrier film 6 from the backing film 4.

Fig. 2 is a drawing (a sectional drawing) illustrating preferable one embodiment of the adhesive skin patch product in the present invention.

In the aspect illustrated in Fig. 2, the support liner 7 is attached to the carrier film 6 and the release liner 5. In a preferred aspect, as illustrated in Fig. 2, the support liner 7 is attached to the carrier film 6 in a center of the support liner 7, and also attached to the release liner 5 in both ends of the support liner 7.

In the aspect illustrated in Fig. 2, the adhesive skin patch product 1 in the present invention has a feature in which both the release liner 5 and the support liner 7 are longer than the adhesive tape 10 and the carrier film 6 that is brought into close contact with the surface of the backing film 4 of the adhesive tape 10, and the adhesive tape 10 and the carrier film 6 are disposed between the release liner 5 and the support liner 7 , both of which are longer than the adhesive tape 10 and the carrier film 6, thereby having an enclosed structure. Thus, employing the release liner 5 and the support liner 7, both of which are longer than the adhesive tape 10, ensures easier releasing of the adhesive 10 from the release liner 5 by grasping and pulling upward an end portion of the support liner 7 as a handle, even though a size of the adhesive tape 10 is small. Furthermore, upon releasing from the release liner 5, grasping the end portion of the support liner 7 as a handle enables a further improvement on easy application of the adhesive tape 10 to the application site, thereby ensuring a further improved handleability of the adhesive skin patch.

A method for using the adhesive skin patch in the present invention (a method for applying to the skin as an applied surface) is, for example, as follows.

First, the adhesive tape 10 (the backing film 4 and the adhesive layer 3) is released from the release liner 5 at the opposite side (side A in Fig. 1) of the side where the non-attached end portion 6a of the carrier film 6 is formed by grasping the support liner 7 and the adhesive tape 10 (the backing film 4 and the adhesive layer 3) together and pulling them upward. When a support liner that is longer than the carrier film 6 is employed for the support liner 7 (Fig. 2), the support liner 7 is released from the release liner 5 at the opposite side (side A in Fig. 2) of the side where the non-attached end portion 6a of the carrier film 6 is formed. Then, pulling the support liner 7 upward as it is with respect to the release liner 5 subsequently releases the adhesive tape 10 (the backing film 4 and the adhesive layer 3) from the release liner 5.

Next, the adhesive layer 3 of the adhesive tape 10 is placed and applied on the applied surface (skin). At this time, when a support liner that is longer than the carrier film 6 is employed for the support liner 7 (Fig. 2), a support adhesive layer 8 of the support liner 7 may be temporarily applied to the applied surface (skin).

Then, by grasping the support liner 7 and the non-attached end portion 6a of the carrier film 6 simultaneously at the side (side B in Fig. 1) where the non-attached end portion 6a of the carrier film 6 is formed and pulling them upward with respect to the applied surface, as well as in the case a support liner that is longer than the carrier film 6 is employed for the support liner 7 (Fig. 2), (in the case where the support adhesive layer 8 of the support liner 7 is applied to the applied surface, the support liner 7 is released from the applied surface, then) pulling the support liner 7 upward with respect to the applied surface at the side (side B in Fig. 2) where the non-attached end portion 6a of the carrier film 6 is formed releases the carrier film 6, which is formed by attaching to the support liner 7, from the backing film 4 of the adhesive tape 10, and the adhesive tape 10 (the backing film 4 and the adhesive layer 3) is applied to the applied surface (skin).

Thus, the support liner 7 is used for facilitating releasing of the adhesive tape 10 from the release liner 5 and applying of the adhesive layer 3 of the adhesive tape 10 to the targeted applied surface. The support liner 7 also plays a role of being used for easily releasing the carrier film 6 formed by being in close contact with the backing film 4 of the adhesive tape 10.

In the adhesive skin patch product 1 in the present invention, a releasing strength P1 of the support adhesive layer 8 of the support liner 7 from the carrier film 6 and a releasing strength P2 of the carrier film 6 from the backing film 4 of the adhesive tape 10 can be adequately set. For example, in a setting of the P1 strength being larger than the P2 strength, when the carrier film 6 is released from the backing film 4 of the adhesive tape 10 after applying the adhesive layer 3 of the adhesive tape 10 to the applied surface (skin), a separation of the support liner 7 and the carrier film 6 can become difficult.

Numerical value range of the releasing strength P1 and the releasing strength P2 is not specifically limited and can be adequately set by considering the handleability of the adhesive skin patch product. For example, by considering a role of the support liner 7, that is, when the carrier film 6 is released from the backing film 4 of the adhesive tape 10 by using the support liner 7, the releasing strength (P1) that prevents releasing of the support liner 7 from the carrier film 6 may be set. Alternatively, when releasing of the carrier film 6 from the backing film 4 of the adhesive tape 10 is not intended, for example, when the adhesive skin patch product 1 is stored or when the release liner is firstly released before use of the adhesive skin patch product 1, the releasing strength (P2) that prevents releasing of the carrier film 6 from the backing film 4 may be set, or the releasing strength (P2) that prevents damaging of the backing film 4 when the support liner 7 is released and that prevents releasing of the adhesive tape 10 applied to the applied surface from the skin may be set. When the above handleability is considered, the lower limit of the P1 and the P2 can be, for example, around 0.03N/15mm, and the upper limit of the P1 and the P2 can be, for example, around 20N/15mm.

Further, each of a releasing strength P3 of the adhesive layer 3 of the adhesive tape 10 from the release liner 5 and a releasing strength P4 of the support adhesive layer 8 of the support liner 7 from the release liner 5 can be set to be smaller than the above P1 and P2 so as to release the support liner 7 and the adhesive tape 10 from the release liner 5 more smoothly and contribute for use.

A relationship of the above-described releasing strengths and the numerical value range of the releasing strength are preferred to be maintained even after sterilization treatment generally carried out after manufacture of medical products. (γ sterilization method, electron beam sterilization method, autoclave sterilization method, ethylene oxide gas sterilization method, etc.)

The following details structures of respective layers that comprise the adhesive skin patch product 1.

It is essential that these structures of respective layers are variously selected by considering the adherability to the applied surface (skin), the operability of the adhesive skin patch, and similar factors.

### Adhesive Skin Patch

In the adhesive skin patch product in the present invention, the above-described adhesive skin patch has a laminated structure of at least four layers (the carrier film, the backing film, the adhesive layer (the adhesive tape is provided with the backing film and the adhesive layer) and the release liner) including the adhesive tape provided with the backing film and the adhesive layer disposed on the surface of one side of the backing film, the release liner attached to the adhesive layer and the carrier film that is brought into close contact with the surface of the backing film that is opposite to the side of the adhesive layer.

### Backing Film

The backing film used for the above-described adhesive tape includes, for example: a paper, such as an impregnated paper, a coated paper, a high-quality paper, a kraft paper, a Japanese paper, and a glassine paper; a plastic film, such as a polyester film, such as polyethylene terephthalate and polybutylene terephthalate, a polyolefin film, such as polyethylene and polypropylene, a polyvinyl chloride film, a polycarbonate film, a polyurethane film, and a cellophane film; a foam; a fabric, such as nonwoven fabric, woven fabric, and knitted fabric made of polyester, polyurethane, polyethylene, and polypropylene; and a laminated body of two or more kinds of these.

Among these, a material flexible enough to come in close contact with the skin and follow a movement of the skin and a material that can inhibit an occurrence of, for example, a skin rash after a long time application is preferred. Among these, the polyester film, the polyurethane film, or the polyolefin film is preferred with the point of excellent followability to the movement (expansion and contraction portion and bending surface) of the skin under the application, and in particular, it is preferred to use the polyolefin film, even more, the polyethylene film.

The backing film can be set to have, for example, its thickness within a range of 5 µm to 1 mm by considering, for example, a physical property, such as a tensile elongation, a tensile strength, and a workability, a touch of application, sealability of an affected part and transferability of each constituent (for example, medical agent) included in the adhesive layer described later to the backing film. The thickness of the backing film is preferred to be 5 µm to 300 µm, more preferably, 10 µm to 100 µm, and yet more preferably, 10 µm to 50 µm. When the thickness of the backing film is smaller (thinner) than 5 µm, a strength and a handleability of the adhesive tape lower to make it difficult to apply to the skin even though the carrier film is disposed. This possibly causes, for example, a contact with other members, a breakage at the expansion and contraction portion and the bending surface of the skin, and releasing from the skin in a short time due to a contact with water, such as bathing. When the thickness of the backing film is too large (exceeding 1 mm), the adhesive tape hardly follow the movement of the skin to easily form a start of releasing at a side edge portion of the adhesive tape. Therefore, the adhesive tape may be released from the skin in a short time and an uncomfortable feeling during the application may be increased.

The backing film is preferred to be set to have, for example, its tensile strength within the range of 1 N/15 mm to 100 N/15 mm and its tensile elongation within the range of 50% to 1500% by considering ensuring a flexibility that can follow the expansion and contraction portion and the bending surface of the skin and an adhesion to the skin. When the tensile strength of the backing film is less than 1 N/15 mm, the strength is low, thus making it hard to follow in the expansion and contraction portion and the bending surface of the skin. Meanwhile, when the tensile strength exceeds 100 N/15 mm, the flexibility lacks on the other hand. Also in this case, following in the expansion and contraction portion and the bending surface of the skin, especially following in expansion becomes difficult, and additionally, a repeated use may cause a skin irritation due to a large resistance caused in expansion. When the tensile elongation is less than 50%, following in the expansion and contraction portion and the bending surface of the skin lacks. When the tensile elongation exceeds 1500%, while following in the expansion and contraction portion and the bending surface of the skin is sufficient, repeated expansion, contraction and bending cause lack of adhesion to the skin, therefore quick following becomes insufficient.

On the backing film, a process, such as a sand-blasting process and a corona treatment, may be performed on a surface where the adhesive layer of the backing film is disposed for an object to improve an anchoring property of the adhesive and the backing film.

Furthermore, in order to make the adhesive tape inconspicuous when being applied to the skin, a film that is high in transparency may be employed or the adhesive tape may be colored into a color tone, such as a skin color, with a colorant, such as pigments, organic pigments, and natural dyes, so as to reduce a mutual difference with the color of the skin when being applied.

The backing film can include additives, such as an antistatic agent and an ultraviolet inhibitor, to an extent that an effect of the present invention is not hindered. As the antistatic agent, for example, surfactant (anionic surfactant, a cationic surfactant, nonionic surfactant, and amphoteric surfactant) is included. The antistatic agent can solve failures of the anchoring property and the step of the backing film.

### Adhesive Layer

As an adhesive that forms the adhesive layer in the adhesive tape of the above-described adhesive skin patch, for example, an acrylic adhesive, a rubber-based adhesive, an urethane adhesive, and a silicone adhesive can be included. One of these adhesives may be used alone or two or more kinds of these adhesives may be mixed and used. Among these, it is preferable to use the rubber-based adhesive from the aspect of, for example, a compatibility with compound components.

The rubber-based adhesive generally includes a rubber-based elastomer, a tackiness agent, and a softener, and as necessary, various kinds of additives, such as a filler and an antioxidizing agent (antioxidant) described later, are further added.

As the rubber-based elastomer, various kinds of thermoplastic elastomers are applicable, including: a thermoplastic block copolymer, such as a styrene-isoprene-styrene copolymer (hereinafter possibly referred to as "SIS"), a styrene-butadienestyrene copolymer (hereinafter possibly referred to as "SBS"), or the hydrogen additives of these, a styrene-ethylene-propylene-styrene copolymer (hereinafter possibly referred to as "SEPS"), a styrene-ethylene-butylene-styrene copolymer (hereinafter possibly referred to as "SEBS"); an ethylene-vinyl acetate copolymer; and an ethylene-alphaolefin copolymer. Among these, a styrene thermoplastic elastomer that is the thermoplastic block copolymer, such as SIS, SBS, SEPS, and SEBS, is preferably used because of an excellent viscosity and aggregating property. Among these, SIS is particularly preferable from the aspects of an adhesive capacity to a human skin and a compatibility with other components. A content of SIS is not particularly restricted, however, when a total mass of the adhesive layer is 100 mass%, SIS is preferably 10 mass% to 50 mass%, and more preferably, 10 mass% to 30 mass%. For SIS, a styrene-isoprene-styrene block copolymer that is commercially available can be used, for example, JSR SIS 5002 (JSR Corporation) can be provided as an example.

As the tackiness agent, for example, a rosin resin, a terpene resin, a coumarone-indene resin, a petroleum-based resin (C5 petroleum resin and C9 petroleum resin), an alicyclic petroleum resin, an alicyclic hydrogenated petroleum resin, a styrene resin, and a dicyclopentadiene resin are provided as examples. A content of the tackiness agent is not particularly restricted, however, when, for example, the total mass of the adhesive layer is 100 mass%, the tackiness agent can be preferably 15 mass% to 55 mass%, and more preferably, 20 mass% to 50 mass%.

As the softener (plasticizer), a petroleum softener, such as a liquid paraffin; a liquid rubber-based softener, such as liquid polyisoprene, polybutene, and polyisobutylene; a dibasic acid ester plasticizer, such as phthalic acid ester and adipate; and other plasticizer, such as polyethylene glycol and a citrate, are provided as examples. Among these, the liquid paraffin can be preferably used because of the excellent compatibility with the rubber-based elastomer and being free from lowering the aggregation. For example, as a commercial product, HICALL (registered trademark, liquid paraffin manufactured by KANEDA Co., Ltd) M series is provided as an example. From the point of viscosity, a content of these softener is, when the total mass of the adhesive layer is 100 mass%, preferably within a range of 25 mass% to 55 mass%, and more preferably 30 mass% to 50 mass%.

Furthermore, the rubber-based adhesive can further contain, as necessary, an additive that is usually combined in the adhesive layer of a percutaneous absorption drug product, such as a pharmacological agent (active ingredient), an antioxidizing agent (antioxidant agent), a filler, a percutaneous absorption accelerator, pigments, a medicine stabilizer, a medicine dissolution improver, and a medicine dissolution inhibitor. Each of these additives can be used alone, or two or more kinds of these additives can be used in combination.

While the thickness of the adhesive layer is not specifically limited, the thickness is appropriately selectable within a range of, for example, 5 µm to 180 µm, preferably, within a range of 10 µm to 150 µm.

### Release Liner

The release liner (also referred to as the release layer or the release paper) used in the above-described adhesive skin patch is released when the adhesive skin patch product is used and disposed to protect a layer (adhesive layer) that comes in contact with the skin until the time of use and prevent the change of properties. For the release liner, one that is commonly used in the technical field of the adhesive skin patch product (adhesive skin patch) can be used. For example, examples can be: a plastic film, such as polyester (such as polyethylene terephthalate, polybutylene terephthalate, and polyethylene naphthalate), polypropylene (such as non-stretched and stretched), polyethylene, polyurethane, polyvinyl chloride, and polystyrene; a paper or a synthetic paper, such as a high-quality paper, a glassine paper, a parchment paper, and a kraft paper; a peeling process paper that is, for example, the above-described plastic film, paper or synthetic paper, and a synthetic fiber being coated with a release agent having a release property, such as a silicone resin and a fluorine resin; an aluminum foil; and a sheet that is colorless or colored, such as a laminated process paper variously laminated these films and sheets and a laminated peeling process paper that is the laminated process paper coated with the release agent.

While the thickness of these release liners is not specifically limited, it is normally within a range of 10 µm to 1 mm, for example, 20 µm to 500 µm, preferably, 40 µm to 200 µm.

In the present invention, the size of the release liner is only needed to be attached to the adhesive layer and longer than the backing film. In addition, the release liner is only needed to be attached to at least a part of the adhesive layer. The release liner may also be attached to a whole surface of the adhesive layer. Furthermore, the release liner is only needed to have a length that can attach to the adhesive layer of the adhesive tape, in a further preferred aspect, the release liner can have a length that can attach even to the support liner in addition to the adhesive layer of the adhesive tape as described later. The release liner is also only needed to be long with respect to at least one direction, such as a longitudinal direction or a short side direction, of the backing film. Moreover, the release liner can be long over a whole dimension of the backing film, such as the longitudinal direction and the short side direction of the backing film.

### Carrier Film

In the adhesive skin patch of the adhesive skin patch product in the present invention, the carrier film is disposed so as to come in close contact with a surface of the backing film that differs from a surface where the adhesive layer is disposed, thereby improving the handleability of the adhesive tape. For example, it is preferred to use various kinds of films made of various kinds of thermoplastic resins, such as polyester, polyurethane, polyethylene, polypropylene, ionomer, polyamide, polyvinyl chloride, polyvinylidene chloride, ethylene vinyl acetate copolymer, thermoplastic polyester, and polytetrafluoroethylene. As the carrier film, one that is in a state of the above-described various kinds of films being laminated to a paper may be used. Among these, it is preferred to use the polyester film from the aspect that the polyester film can make the handleability of the adhesive tape preferable.

The carrier film is preferred to have its thickness thick or be made of a material with a strong stiffness so as to achieve the object to improve the handleability of the adhesive tape. The thickness of the carrier film is normally 10 µm to 500 µm, preferably, 20 µm to 250 µm. When the thickness of the carrier film is less than 10 µm, the backing film of the adhesive tape and the carrier film are not sufficiently brought into close contact. When the thickness of the carrier film exceeds 500 µm, while the adhesion with the backing film of the adhesive tape is sufficient and the operability is improved, the rigidity of the carrier film is excessively increased. Therefore, for example, when the adhesive layer is applied to the skin after releasing the release liner at the point of use, the followability to the skin lacks and the attachability in the curved surface portion and similar portions is insufficient.

For the carrier film, it is preferable to employ one with the tensile strength within a range of 3 N/10 mm to 200 N/10 mm and the tensile elongation within a range of 10% to 1500%. When the tensile strength is less than 3 N/10 mm, the sufficient rigidity is not obtained. The lack of stiffness possibly leads to an insufficient obtainment of an effect of improved handleability of the adhesive tape. On the other hand, when the tensile strength exceeds 200 N/10 mm, while the handleability of the adhesive tape improves, the rigidity is excessively increased to cause an insufficient followability to the skin and attaching force to the curved surface portion when the adhesive tape is applied. Furthermore, when the tensile elongation of the carrier film is less than 10%, the following of the carrier film to the backing film of the adhesive tape is difficult when, for example, applying to the curved surface portion. When the tensile elongation exceeds 1500%, while the following to the backing film of the adhesive tape is sufficient, when the carrier film extends large compared with the backing film of the adhesive tape, lifting is generated at an interface between the backing film and the carrier film, thereby possibly causing the handleability of the adhesive tape to degrade on the contrary.

In the adhesive skin patch of the adhesive skin patch product in the present invention, the carrier film has a feature in which, in the end portion on one side of the carrier film, the non-attached end portion that is not in close contact with the surface of the backing film of the adhesive tape and separated from the backing film is formed. Easy releasing of the carrier film, which is formed by attaching to the support liner, from the backing film of the adhesive tape is ensured by grasping and pulling upward the support liner and the non-attached end portion together from the side where the non-attached end portion is disposed or, when the support liner has a length that extends long with respect to the outer edge of the carrier film in the side where the non-attached end portion is disposed, by grasping and pulling upward only the support liner from the side where the non-attached end portion is disposed.

The non-attached end portion is set to be of a size (area) so as to improve its releasability when the carrier film is released from the backing film. However, the non-attached end portion is set to be of a size (area) so as not to be released from the backing film when releasing of the carrier film from the backing film is not intended, for example, when the adhesive skin patch is stored or when the release liner is firstly released before use of the adhesive skin patch. For example, a proportion of the area of the non-attached end portion to the whole area of the carrier film is preferred to be defined between 1% and 50%.

### Support Liner

In the adhesive skin patch product in the present invention, the above-described support liner has a laminated structure of at least two layers including the tape backing layer and the support adhesive layer disposed on a surface of the tape backing layer.

The support liner is preferred to be longer than the adhesive tape and the carrier film which is formed by being in close contact with the backing film of the adhesive tape. In a particularly preferred aspect, the support liner is preferred to have a size formed by attaching to the carrier film of the adhesive skin patch and attaching to the release liner of the adhesive skin patch.

In addition, the attaching position of the support liner with respect to the carrier film of the adhesive skin patch is not particularly limited. For example, the support liner can attach to the carrier film in a center of the support liner, or can attach to the carrier film in a portion from the center to a side close to the outer side of the support liner. Furthermore, when the support liner is formed by attaching also to the release liner in addition to the carrier film, the attaching position of the support liner with respect to the release liner of the adhesive skin patch is not particularly limited. That is, at least a part of the support liner needs to be attached to the release liner. For example, the support liner can be considered to attach to the release liner in one end or both ends of the support liner, when viewed form a longitudinal direction or a short side direction of the support liner.

In a preferred aspect, the support liner can be one that has a size (length) in which the support liner can attach to the carrier film of the adhesive skin patch in the center of the support liner and the support liner can attach to the release liner of the adhesive skin patch in both ends of the support liner.

By employing the support liner in the adhesive skin patch product in the present invention, the support liner can further facilitate releasing of the adhesive tape from the release liner and releasing of the carrier film from the backing film of the adhesive tape. The support liner can further improve the operability when the adhesive layer of the adhesive tape is applied to the applied surface.

### Tape Backing Layer

As the tape backing layer of the above-described support liner, one that is identical to the backing film in the adhesive tape of the above-described adhesive skin patch can be employed. Among these, the polyethylene film, a polyolefin nonwoven fabric, a cyclic polyolefin film, and the polyester film are preferred from the aspect of making the handleability of the adhesive skin patch product satisfactory, in particular, the polyethylene film and the polyolefin nonwoven fabric are preferred.

The tape backing layer can be set to have, for example, its thickness within a range of 25 µm to 500 µm by considering the physical property, such as the tensile elongation, the tensile strength, and the workability.

The tape backing layer is preferred to have an appropriate flexibility so as to improve the handleability of the adhesive skin patch and the adhesive tape, for example, 60 mm or more of bending resistance is preferred.

On a surface on the side where the support adhesive layer of the tape backing layer is not disposed, usage instructions of the adhesive skin patch of the present invention, for example, releasing procedures and releasing instructions of the release liner, kinds (kinds of combined active ingredients) of the adhesive tape, can be specified by means of printing and similar means.

### Support Adhesive Layer

As the support adhesive layer of the above-described support liner, one that is identical to the adhesive layer in the adhesive tape of the above-described adhesive skin patch can be employed. For example, the acrylic adhesive, the rubber-based adhesive, the urethane adhesive, and the silicone adhesive can be provided as examples. It is preferable to use the rubber-based adhesive from the aspect of the compatibility with the compound components.

While the thickness of the support adhesive layer of the support liner is not particularly limited, the thickness is appropriately selectable within a range of, for example, 5 µm to 180 µm, preferably, 10 µm to 150 µm.

### Manufacturing Method of Adhesive Skin Patch

While a manufacturing method of the adhesive skin patch product in the present invention is not particularly limited, methods commonly performed in the conventional adhesive skin patch products (adhesive skin patches) or adhesive tapes can be employed in appropriate combination. In a preferred aspect, as one step of manufacturing the adhesive skin patch product, a step of laminating the backing film on the carrier film by extruding a material that comprises the backing film of the adhesive tape to the carrier film or a step of simultaneously manufacturing (laminating) the backing film of the adhesive tape and the carrier film is performed. By doing the above, it is preferred to produce a laminated structure comprising the carrier film and the backing film of the adhesive tape. These laminating methods can adopt the conventionally known methods that include, such as a method for extruding a molten resin material (backing film material or carrier film material) from a nozzle, for example, a T-die method and an inflation method, a method for co-extruding two kinds of molten resin materials (backing film material and carrier film material) and a method for rolling two kinds of films (backing film and carrier film) with a pressure roll, for example, by a calendaring method.

In a preferable aspect of the adhesive skin patch in the present invention, a laminated body comprising the backing film of the adhesive tape and the carrier film is produced by firstly extruding a molten resin material that comprises the backing film of the adhesive tape to the carrier film. This laminated body includes a part in which the backing film and the carrier film are not in close contact with each other to form a non-attached end portion in a completed product (the adhesive skin patch). Next, a material of the adhesive layer in the adhesive tape is separately coated on a release paper. Followingly, a surface of the backing film in the laminated body is laminated on the coated surface, and a structure of the adhesive tape and the adhesive skin patch becomes completed.

Meanwhile, a material of the support adhesive layer in the support liner is coated on a release paper (not included in a structure of the adhesive skin patch product in the present invention), and then the tape backing layer is laminated on the coated surface. A structure of the support liner thus becomes completed on the release paper.

Furthermore, by releasing the support liner from the release paper and attaching it to the carrier film of the adhesive skin patch, and by attaching the support liner to the carrier film and to the release liner in a preferred aspect, a structure of the adhesive skin patch product becomes completed.

### [WORKING EXAMPLES]

The following indicates and further specifically describes working examples of the present invention, however, the present invention is not limited to these, and various kinds of applications are allowed within the range not departing the technical idea of the present invention. Methods for measuring characteristics and physical properties of each layer provided in the adhesive skin patch and the support liner of the adhesive skin patch product in the present invention are as follows.

### Thickness

The thickness of the tape backing layer and the support adhesive layer in the support liner, the adhesive skin patch and the carrier film provided in the adhesive skin patch, the backing film and the adhesive layer in the adhesive tape, as well as the release liner was measured by dial thickness gauge.

### Handleability

Evaluations of the handleabilities of the product in the present invention were evaluated by the following method. That is, when applying the product of the present invention to the cheeks of 7 subjects of adult males and females, the handleabilities were evaluated with respect to the easiness of releasing the adhesive tape from the release liner (easiness of releasing the adhesive tape from the release liner by using the support liner in Working Examples and Comparative Example 1), the followability to the skin in the adhesive skin patch product (the support liner, the carrier film and the adhesive tape in Working Examples and Comparative Example 1, the carrier film and the adhesive tape in Comparative Example 2) when the release liner is released and the easiness of releasing the carrier film, according to the scoring below.

⊚ Average score is no less than 4 (excellent in handleability of the product)
○ Average score is no less than 3 and less than 4 (good in handleability of the product)
Δ Average score is no less than 2 and less than 3 (lack of handleability of the product)
× Average score is less than 2 (inadequate in handleability of the product)

### Easiness of Releasing Adhesive Tape from Release Liner

- 5:: Easy to release
- 4:: Slightly easy to release
- 3:: Neutral
- 2:: Slightly difficult to release
- 1:: Difficult to release

### Followability to Skin in Adhesive Skin Patch Product when Release Liner is Released

- 5:: Good in followability
- 4:: Slightly good in followability
- 3:: Neutral
- 2:: Slightly bad in followability
- 1:: Bad in followability

### Easiness of Releasing Carrier Film

- 5:: Easy to release
- 4:: Slightly easy to release
- 3:: Neutral
- 2:: Slightly difficult to release
- 1:: Difficult to release

### Example 1

### Manufacturing Method of Adhesive Skin Patch

A coating liquid to form an adhesive layer with a solid content of 57 mass% was prepared by mixing 100 pts. mass of a styrene-isoprene-styrene block copolymer (JSR SIS5002 manufactured by JSR Corporation), 250 pts. mass of a terpene resin (YS resin manufactured by YASUHARA CHEMICAL CO., LTD.) as a tackifying resin, and 350 pts. mass of a liquid paraffin (HICALL (registered trademark) M-352 manufactured by KANEDA Co., Ltd) as a softener, and dissolving them in a mixed solvent of toluene/ acetone = 7/3 (mass ratio).

This coating liquid was applied using a bar coater over one surface of a release liner (a silicone processed polyethylene terephthalate film, 75 µm of thickness) so as to have a thickness of 20 µm after drying. The coating liquid was then dried, and a laminated body A comprising the release liner and the adhesive layer was obtained.

Subsequently, by extruding a molten resin material (polyethylene) that comprises a backing film of an adhesive tape to a carrier film made of the polyethylene terephthalate, a laminated body B comprising the backing film made of the polyethylene film with 15 µm of thickness and the carrier film made of the polyethylene terephthalate with 25 µm of thickness was obtained.

The laminated body A and the laminated body B were laminated such that the adhesive layer of the above-described laminated body A and the backing film of the above-described laminated body B were overlapped each other. Then, a non-attached end portion was formed by partly releasing the carrier film and the backing film made of the polyethylene film at an end portion of the laminated body A. The non-attached end portion was formed to have an area less than 50% of a whole area of the carrier film when the carrier film being as an adhesive skin patch product, which will be described later. Only the adhesive tape provided with the carrier film was half-cut (the release liner was not cut) to form a circular shape with ϕ 27 mm of diameter such that the non-attached end portion was included.

### Manufacturing Method of Support Liner

A coating liquid to form an adhesive layer with a solid content of 30 mass% was prepared by mixing 100 pts. mass of a styrene-isoprene-styrene block copolymer (JSR SIS5002 manufactured by JSR Corporation) and 25 pts. mass of a terpene resin (YS resin manufactured by YASUHARA CHEMICAL CO., LTD.) as a tackifying resin, and dissolving them in a mixed solvent of toluene/hexane = 8/2 (mass ratio).

This coating liquid was applied using a bar coater over one surface of a release paper (a silicone processed polyethylene terephthalate film, 25 µm of thickness) on which a release layer was to be formed, so as to have a thickness of 50 µm after drying. Next, the coating liquid was dried. After a laminated body comprising the release layer and the adhesive layer was obtained, a tape backing layer made of a polyethylene film with 80 µm of thickness was laminated on the adhesive layer to provide a support liner.

### Manufacturing Method of Adhesive Skin Patch Product

The release paper attached to the above-prepared support liner was released, and the support liner was laminated on the carrier film (the adhesive tape) and on the release liner in a peripheral area of the part where the adhesive tape was attached (the part where the adhesive tape was not attached) such that the support liner covered the circular shaped adhesive tape with ϕ 27 mm of diameter. This was then cut into a rectangle of 14 mm × 47 mm to produce the adhesive skin patch (the adhesive skin patch product) to which the support liner was attached as illustrated in Fig. 2, and its handleability was evaluated. The results of evaluation are shown in Table 1.

### Example 2

The adhesive skin patch product was produced in the same manner as Example 1 except that the tape backing layer of the support liner was a polyethylene terephthalate film with 50 µm of thickness, and its handleability was evaluated. The results of evaluation are shown in Table 1.

### Example 3

The adhesive skin patch product was produced in the same manner as Example 1 except that the tape backing layer of the support liner was a cyclic polyolefin film with 75 µm of thickness, and its handleability was evaluated. The results of evaluation are shown in Table 1.

### Example 4

The adhesive skin patch product was produced in the same manner as Example 1 except that the tape backing layer of the support liner was a blend film of polyethylene with 65 µm of thickness and cyclic polyolefin (blending ratio = 1: 1), and its handleability was evaluated. The results of evaluation are shown in Table 1.

### Example 5

The adhesive skin patch product was produced in the same manner as Example 1 except that the tape backing layer of the support liner was a polyolefin nonwoven fabric with 440 µm of thickness, and its handleability was evaluated. The results of evaluation are shown in Table 1.

### Example 6

The adhesive skin patch product was produced in the same manner as Example 1 except that the adhesive tape and the support liner were same in size, both of which being in a form of almost a strip with a size of 14 mm x 27 mm (see Fig. 1), and its handleability was evaluated. The results of evaluation are shown in Table 1.

### Comparative Example 1

The adhesive skin patch product was produced in the same manner as Example 1 except that the carrier film did not include one end portion preventing close contact with the backing film, that is, the non-attached end portion was not formed, and its handleability was evaluated. The results of evaluation are shown in Table 1.

### Comparative Example 2

A coating liquid to form an adhesive layer with a solid content of 57 mass% was prepared by mixing 100 pts. mass of a styrene-isoprene-styrene block copolymer (JSR SIS5002 manufactured by JSR Corporation), 250 pts. mass of a terpene resin (YS resin manufactured by YASUHARA CHEMICAL CO., LTD.) as a tackifying resin, and 350 pts. mass of a liquid paraffin (HICALL (registered trademark) M-352 manufactured by KANEDA Co., Ltd) as a softener, and dissolving them in a mixed solvent of toluene/ acetone = 7/3 (mass ratio).

This coating liquid was applied using a bar coater over one surface of a release paper (a silicone processed polyethylene terephthalate film, 75 µm of thickness) on which a release layer was to be formed, so as to have a thickness of 20µm after drying. The coating liquid was then dried, and a laminated body E comprising the release layer and the adhesive layer was obtained.

Subsequently, by extruding a molten resin material (polyethylene) that comprises a backing film of an adhesive tape to a carrier film made of the polyethylene terephthalate, a laminated body F comprising the backing film made of the polyethylene film with 15 µm of thickness and the carrier film made of the polyethylene terephthalate with 25 µm of thickness was obtained.

The laminated body E and the laminated body F were laminated such that the adhesive layer of the above-described laminated body E and the backing film of the above-described laminated body F were overlapped each other. Then, a non-attached end portion was formed by partly releasing the carrier film and the polyethylene film at an end portion. The non-attached end portion was formed to have an area less than 50% of a whole area of the carrier film when the carrier film being as an adhesive skin patch, which will be described later. After cutting to include the non-attached end portion, the adhesive skin patch in a form of almost a strip with a size of 14 mm x 27 mm (a support liner was not cut, the non-attached end portion was included) was manufactured and its handleability was evaluated. Table 1 indicates results of evaluation of the handleability.

**[Table 1]**

| Evaluation Items | Working Examples | | | | | | Comparative Examples | |
|---|---|---|---|---|---|---|---|---|
| | 1 | 2 | 3 | 4 | 5 | 6 | 1 | 2 |
| Easiness of Releasing from Liner | ⊚ | ⊚ | ⊚ | ⊚ | ⊚ | ○ | ⊚ | Δ |
| Followability to Skin | ⊚ | ○ | ○ | ○ | ⊚ | ⊚ | ⊚ | ⊚ |
| Easiness of Releasing Carrier Film | ⊚ | ⊚ | ⊚ | ⊚ | ⊚ | ○ | × | ⊚ |

As Table 1 indicates, in the adhesive skin patch products of Working Examples 1 to 5, the evaluations were excellent in the evaluation items of both Easiness of Releasing from Liner and Easiness of Releasing Carrier Film, and the evaluation was good in Followability to Skin. Therefore, almost satisfactory results were obtained with respect to the handleability of the product. Additionally, in Example 6 where the adhesive tape and the support liner were same in size, the result being good in the handleability of the product was obtained by attaching the support liner and disposing the non-attached end portion.

Meanwhile, in Comparative Example 1, the evaluation being inadequate in the handleability of the product was obtained due to the difficulty in releasing the carrier film even though the support liner was disposed. In Comparative Example 2, the result lacking the handleability of the product was obtained due to the difficulty in releasing the adhesive tape from the liner.

### [EXPLANATION FOR REFERENCES]

- 1:: Adhesive skin patch product
- 2:: Adhesive skin patch
- 3:: Adhesive layer
- 4:: Backing film
- 5:: Release liner
- 6:: Carrier film
- 6a:: Non-attached end portion
- 7:: Support liner
- 8: Support adhesive layer
- 9:: Tape backing layer
- 10:: Adhesive tape

## Claims

1. An adhesive skin patch product, comprising:
an adhesive skin patch;
a support liner that covers an upper side of the adhesive skin patch,
wherein the adhesive skin patch comprises:
an adhesive tape in a form of an approximately strip that includes a backing film and an adhesive layer disposed on a surface of one side of the backing film;
a release liner that is attached to the adhesive layer and is longer than the backing film; and
a carrier film that is brought into close contact with a surface of the backing
film that is opposite to a side of the adhesive layer,
wherein the support liner comprises a tape backing layer and a support adhesive layer disposed on a surface of the tape backing layer, the support liner being attached to the carrier film, and
wherein the carrier film includes one end portion preventing close contact with a surface of the backing film of the adhesive tape, the one end portion being released from the backing film to form a non-attached end portion.

2. The adhesive skin patch product according to claim 1, wherein the support liner is attached to the carrier film and the release liner.

3. The adhesive skin patch product according to claim 2, wherein the support liner is attached to the carrier film in a center of the support liner, and attached to the release liner in both ends of the support liner.

4. The adhesive skin patch product according to any one of claims 1 to 3, wherein the backing film of the adhesive tape is made of a polyolefin film, and the carrier film is made of a polyester film.

5. The adhesive skin patch product according to any one of claims 1 to 4, wherein the adhesive layer of the adhesive tape and the support adhesive layer of the support liner include a rubber-based adhesive.

6. The adhesive skin patch product according to any one of claims 1 to 5, wherein a proportion of an area of the non-attached end portion to a whole area of the carrier film is defined between 1% and 50%.
